# EUROPÄISCHE PATENTSCHRIFT

(11) **EP 0 655 904 B1**
(45) Veröffentlichungstag und Bekanntmachung des Hinweises auf die Patenterteilung: **27.01.1999**
(21) Anmeldenummer: 94921569.3
(22) Anmeldetag: 23.06.1994
(51) Int. Cl.: A61K 7/06, A61K 7/48

(54) **VERWENDUNG VON ALKANDIOLEN IN KOSMETISCHEN PRODUKTEN**
USE OF ALKANE DIOLS IN COSMETICS
UTILISATION D'ALCANEDIOLS DANS DES PRODUITS COSMETIQUES

(30) Priorität: 23.06.1993 DE 4320744
(43) Veröffentlichungstag der Anmeldung: 07.06.1995
(73) Patentinhaber: Dragoco Gerberding & Co Aktiengesellschaft, D-37603 Holzminden (DE)
(72) Erfinder: SCHRADER, Karlheinz, D-37639 Bevern (DE)
(74) Vertreter: Eikenberg, Kurt-Rudolf, Dr. Dipl.-Chem.
(86) Internationale Anmeldenummer: DE9400770
(87) Internationale Veröffentlichungsnummer: WO9501151

(56) Entgegenhaltungen:
- EP-A- 0 336 803
- DE-C- 885 602
- FR-A- 2 254 636
- US-A- 3 970 759
- PATENT ABSTRACTS OF JAPAN vol. 6, no. 57 (C-98) (935) 14. April 1982 & JP,A,57 002 212 (POLA KASEI KOGYO K.K.) 7. Januar 1982

## Beschreibung

Die Haut ist ein wichtiges, zugleich aber auch empfindliches menschliches Organ, dessen Pflege für das physische und psychische Wohlbefinden unerläßlich ist. Es sind zahlreiche Hautpflegemittel entwickelt worden, die als Cremes, Lotionen, Öle oder Gele angeboten werden und spezielle hautpflegende Wirkstoffe enthalten.

Neben anderen Zielen der Hautpflege ist die Regelung des Wasserhaushalts der Haut von nicht unbeträchtlicher Bedeutung, denn die Haut neigt, nicht zuletzt bedingt durch Witterungs- und Umwelteinflüsse, zur Schädigung durch Austrocknung. Als feuchtigkeitsregulierende Wirkstoffe werden zumeist Gemische eingesetzt, deren Hauptbestandteile häufig Harnstoff, freie Aminosäuren, Pyrolidoncarbonsäure und Lactat in unterschiedlichen Mengenanteilen sind.

Es wurde nun gefunden, daß 1,2-Alkandiole mit 5 bis 10 C-Atomen eine sehr gute hautbefeuchtende Wirkung aufweisen. Da sich diese Stoffe zugleich als hautverträglich und physiologisch unbedenklich erwiesen haben, können sie auch in höherer Dosierung als feuchtigkeitsregulierende Wirkstoffe in kosmetischen Produkten eingesetzt werden.

Die Erfindung betrifft die Verwendung von 1,2-Alkandiolen mit 5 bis 10 C-Atomen in kosmetischen Produkten, wobei die Verwendung von geradkettigen 1,2-Alkandiolen mit 5 bis 7 C-Atomen bevorzugt wird. Die Einsatzmenge dieser Alkandiole kann im Bereich von 1 - 10 Gew.% liegen und beträgt vorzugsweise 2 - 5 Gew.%, jeweils bezogen auf das kosmetische Produkt.

1,2-Alkandiole (C₅ - C₁₀) sind als Zwischenprodukte für die Herstellung verschiedener Syntheseprodukte bekannt. In kosmetischen Produkten sind sie bislang noch nicht eingesetzt worden, und auch ihre hautbefeuchtende Wirkung ist bislang noch nicht beschrieben worden. Lediglich kurzkettige Polyole mit C₂ - C₄ werden in der Kosmetik verwendet, und zwar durch Wasserbindung im kosmetischen Produkt. Zur Hautfeuchtigkeitsregulierung können sie nicht vorteilhaft verwendet werden. Es war daher überraschend und nicht vorhersehbar, daß die Alkandiole mit 5 - 10 C-Atomen in kosmetischen Produkten als hautfeuchtigkeitsregulierende Wirkstoffe geeignet sind.

Die Erfindung wird nachfolgend in einem Ausführungsbeispiel näher erläutert. Dem Ausführungsbeispiel liegt eine Öl-in-Wasser-Emulsion (O/W-Creme) zugrunde, und als Wirkstoff wurde 1,2-Pentandiol gewählt, weil es sich gegenüber den längerkettigen Diolen durch eine praktisch unbegrenzte Mischbarkeit mit Wasser auszeichnet.

Es wurden zwei Proben der O/W-Creme hergestellt, von denen die eine (erfindungsgemäß) 5 Gew.% 1,2-Pentandiol enthielt, während in der anderen Probe (Placebo) das Pentandiol durch Wasser ersetzt war. Die Zusammensetzung der beiden Proben ergibt sich aus der Tabelle 1.

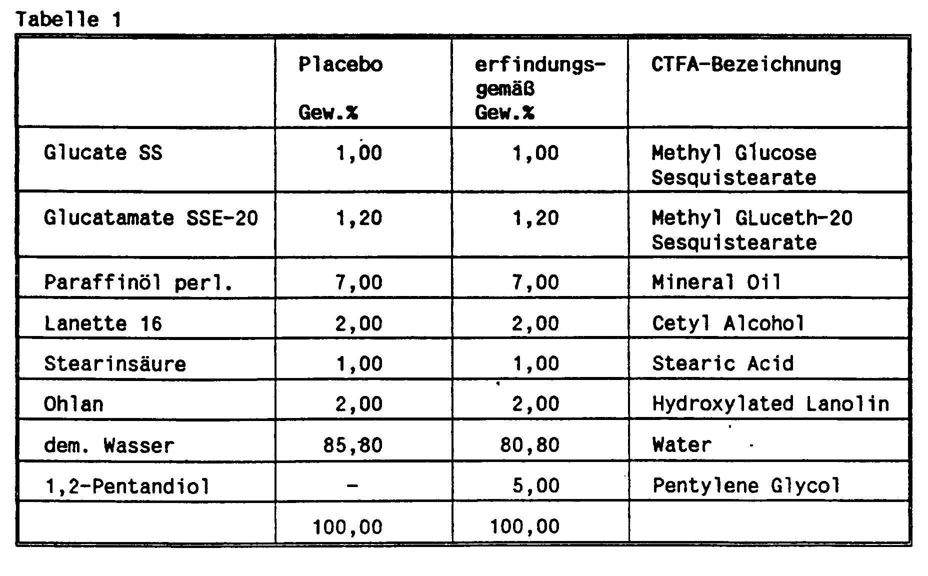

Sodann wurde die Beeinflussung der Hautfeuchtigkeit durch diese beiden Proben der O/W-Creme ermittelt, und zwar mit Hilfe von 20 weiblichen Testpersonen. Diese Testpersonen applizierten 14 Tage lang zweimal täglich die eine der beiden Proben auf bestimmte Testfelder am Unterarm, wobei zum Vergleich ein Leerfeld unbehandelt blieb. Um deutlichere Ergebnisse auszuarbeiten, wurden winterliche Testbedingungen mit Hilfe einer leichten Vorschädigung der Haut am volaren Unterarm simuliert. Dazu wuschen die Testpersonen vor jeder Anwendung der Proben die Unterarme mit einer 2 %igen Na-Laurylsulfatlösung. Diese Prozedur führt bei Leerfeldern zur Austrocknung der Haut, es kommt auch zu einer leichten Aufrauhung. Geprüft wird, wie weit diese negativen Wirkungen von den Produkten ausgeglichen werden. Im Vergleich zum Leerfeld ergeben sich dann aussagefähige Befeuchtungswerte.

Zur Messung der Hautfeuchte diente ein vollautomatisch arbeitendes Gerät vom Typ Corneometer CM 820 PC. Dieses Gerät arbeitet mit einer kapazitiven Meßmethode und nutzt die Tatsache aus, daß sich die Dielektrizitätskonstante des Wassers von den meisten anderen Stoffen deutlich unterscheidet.

Ein entsprechend geformter Meßkondensator (Sensor) reagiert auf die zu untersuchenden Hautfelder mit unterschiedlichen Kapazitätsänderungen (je nach Wassergehalt). Diese Kapazitätsänderungen des Sensors werden vom Gerät vollautomatisch zu einem digitalen Meßwert verarbeitet. Es besteht zwischen dem Meßobjekt und dem Meßgerät keine leitende (galvanische) Verbindung; deswegen fließt durch das Meßobjekt fast kein Strom. Eigenschaften wie Ionenleitfähigkeit und Polarisationseffekte bleiben also praktisch ohne Einfluß auf das Meßergebnis. Der fast trägheltslose "Anpassungsverlauf" der Elektronik an die vorgefundenen Feuchtigkeitsverhältnisse ermöglicht eine sehr schnelle Messung und eine weitgehende Ausschaltung der Beeinflussung der Ergebnisse durch unwillkürliche Bewegungen oder Feuchtigkeitsstau während der Messung.

Der Meßsensor ist quadratisch ausgebildet. Seine spezialglasbeschichtete aktive Stirnfläche ist axial beweglich und hat einen Hub von wenigstens 3 mm. Das Meßprinzip verlangt ein planes Anliegen an die Stirnfläche bei konstantem Andruck. Um dies möglichst reproduzierbar zu gewährleisten, wurde die Stirnseite des Sensors sehr klein (7 x 7 mm) ausgelegt. Der innere bewegliche Teil - die aktive Stirnfläche - wurde durch eine Feder mit jeweils 3,5 N auf die zu messende Stelle der Haut gedrückt. Nach einer Sekunde wurde der Meßwert angezeigt.

Die Testpersonen wurden angewiesen, drei Tage vor Beginn der Prüfung keine Kosmetika auf den Unterarmen anzuwenden. Am Beginn der Prüfung wurden die Testpersonen 45 min lang bei einer Raumtemperatur von 22° C und 60 % relativer Luftfeuchte einklimatisiert, und dann wurde der Ausgangswert der Hautfeuchtigkeit sowohl im Leerfeld als auch in drei Testfeldern gemessen. Die Werte der Testfelder wurden pro Person ermittelt.

Nach Beendigung der 14-tägigen Anwendung der Proben wurde eine Pause von ca. 12 h eingehalten, in der auch die Laurylsulfatbehandlung ausgesetzt wurde. Am 15. Tag erfolgte dann eine Messung des Endwertes der Hautfeuchtigkeit, wobei in gleicher Weise vorgegangen wurde wie bei der Messung des Ausgangswertes.

Die Meßergebnisse sind in der Tabelle 2 niedergelegt. Diese Tabelle enthält die unmittelbar gemessenen Ausgangs- und Endwerte (in Skalenteilen) sowie deren prozentuale Änderungen in der unkorrigierten Fassung (Differenz der Meßwerte) und in der korrigierten Fassung (Differenz nach Leerfeld-Abzug, um die Veränderung des Basiswertes der Hautfeuchtigkeit während der Prüfzeit zu eliminieren). Weiterhin ist die Signifikanz (Irrtumswahrscheinlichkeit) angegeben.

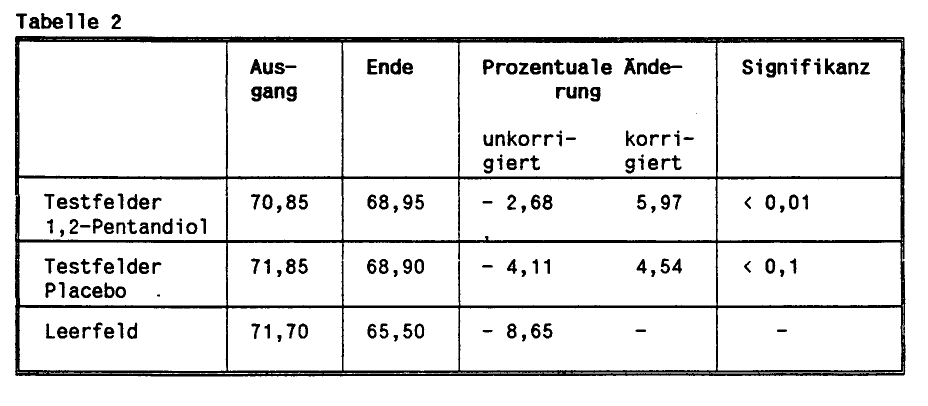

Für die Bewertung der Meßergebnisse kommt es darauf an, ob zwischen dem Ausgangswert und dem Endwert (in der korrigierten Fassung) 12 Stunden nach der letzten Anwendung der Proben ein signifikanter Unterschied in derHautfeuchtigkeit besteht. Tatsächlich belegen die Meßergebnisse, daß ein Gehalt von 5 Gew.% 1,2-Pentandiol in der gewählten O/W Creme trotz der simulierten ungünstigen Witterungsbedingungen eine deutliche Verbesserung der Hautfeuchtigkeit bewirkt hat, und zwar um rund 30 %, bezogen auf die Placebowirkung.

## Patentansprüche

1. Verwendung von 1,2-Alkandiolen mit 5 - 10 C-Atomen als Hautbefeuchtungsmittel in kosmetischen Produkten.

2. Verwendung nach Anspruch 1, wobei es sich um geradkettige Alkandiole mit 5 bis 7 C-Atomen handelt.

3. Verwendung nach einem der vorhergehenden Ansprüche, wobei die Alkandiole in einer Menge von 1 - 10 Gew.% und vorzugsweise 2 - 5 Gew.% zum Einsatz kommen, jeweils bezogen auf das kosmetische Produkt.

## Claims

1. Use of 1,2-alkane diols with 5 to 10 carbon atoms as skin moisturising agents in cosmetic products.

2. Use according to claim 1, wherein straight chain alkane diols with 5 to 7 carbon atoms are used.

3. Use according to either of the preceding claims, wherein the alkane diols are present in a mixture in an amount of 1 to 10% by weight, and especially 2 to 5% by weight, respectively relative to the cosmetic product.

## Revendications

1. Utilisation d'1,2-alcandioles avec 5 à 10 atomes de carbone comme substance d'humidification de la peau dans des produits cosmétiques.

2. Utilisation selon la revendication 1 pour laquelle il s'agit d'alcandioles en chaîne linéaire avec 5 à 7 atomes de carbone.

3. Utilisation selon l'une des revendications précédentes, les alcandioles étant utilisés en une quantité d'1 à 10 % en poids, et de préférence de 2 à 5 % en poids, respectivement par rapport au produit cosmétique.
